# EUROPEAN PATENT APPLICATION

(11) **EP 4 001 804 A1**
(43) Date of publication of application: **25.05.2022**
(21) Application number: 21208843.9
(22) Date of filing: 17.11.2021
(51) Int. Cl.: F25D 17/04, F25D 17/06

(54) **REFRIGERATOR**

(30) Priority: 19.11.2020 KR 20200155755
(71) Applicant: LG Electronics Inc., Seoul 07336 (KR)
(72) Inventor: CHOI, Jaegeon, 08592 Seoul (KR)
(74) Representative: Ter Meer Steinmeister & Partner

(57) **Abstract**

A refrigerator includes a cabinet having a storage space, an evaporator provided at one side of the storage space, and a grill pan assembly forming an inner surface of the storage space, and including a fluid passage to guide cold air, which is generated from the evaporator, to the storage space, and a discharge port to discharge the cold air to the storage space. The fluid passage may include a first fluid passage to receive the cold air supplied from the evaporator, and a second fluid passage communicating with an outlet of the first fluid passage and split into a plurality of fluid passages. A deodorizing device is provided in the first fluid passage to remove odor, and the cold air passing through the first fluid passage is split at the second fluid passage after passing through the deodorizing device.

## Description

### BACKGROUND

The present disclosure relates to a refrigerator.

In general, a refrigerator, which is a home appliance to store foods at a lower temperature in an internal storage space shielded by a refrigerator door, cools the internal storage space by using cold air produced through heat exchange with a refrigerant circulating in a refrigeration cycle, thereby storing the foods in the optimal state.

As described above, the refrigerator has been gradually enlarged and multi-functioned, with the change in food life and the improvement in product quality. In addition, recently, refrigerators having various structures and convenience devices have been launched, based on user convenience.

In particular, a refrigerator having a deodorizing device has been introduced to improve hygiene inside the refrigerator and resolve the dissatisfaction of a user with odor inside the refrigerator.

In general, a deodorizing device has a structure disposed in a fluid passage of cold air to remove odor from the air circulating inside the refrigerator. Korean Unexamined Publication Patent No. 10-1999-0080055 discloses a refrigerator having a structure in which a deodorant to be suctioned into an evaporator is placed in a return duct.

However, in the refrigerator having the above structure, the deodorant and the structure having the deodorant are not exposed to the outside, which degrades an assembling property and service performance for maintenance.

In particular, when the replacement of the deodorant is required or when a foreign substance is caught in the relevant structure, so the refrigerator needs to be cleaned, or an action needs to be taken, the above works are not easy.

### SUMMARY

An object of the present disclosure is to provide a refrigerator capable of removing odor.

Another object of the present disclosure is to provide a refrigerator capable efficiently deodorizing cold air and then splitting/distributing the deodorizing cold air and supplying the same to the storage space.

Yet another object of the present disclosure is to provide a refrigerator capable of removing odor without an additional fluid passage for deodorizing.

Still another object of the present disclosure is to provide a refrigerator having a deodorizing device mounted in a discharge fluid passage through a grill pan, thereby improving assembly workability and the convenience in maintenance.

Another object of the present disclosure is to provide a refrigerator having a deodorizing device mounted in a discharge fluid passage, thereby uniformly providing the flow of air, which is sterilized and deodorized, without degrading refrigerating performance.

One or more objects of the present technique are achieved by the features of independent claim(s).

According to a first aspect of the present technique a refrigerator is presented. The refrigerator includes a cabinet having a storage space; an evaporator configured to generate cold air; a grill pan assembly forming a surface defining the storage space; and a deodorizing device to remove odor from the cold air. The grill pan assembly includes a fluid passage configured to guide cold air generated from the evaporator to the storage space, and at least one, i.e. one or a plurality of discharge ports, to discharge the cold air from the fluid passage into the storage space. The fluid passage includes a first fluid passage for receiving the cold air from the evaporator, and a second fluid passage communicating with an outlet of the first fluid passage for receiving the cold air from the first fluid passage. The second fluid passage comprises a plurality of fluid passages configured to split the cold air receiving from the first fluid passage and to guide the cold air so split to the storage space via the at least one discharge port. The deodorizing device is suspended in the first fluid passage in a flow path of the cold air flowing therethrough and upstream of plurality of fluid passages of the second fluid passage.

According to a second aspect of the present technique, a refrigerator may include a cabinet having a storage space, an evaporator provided at one side of the storage space, and a grill pan assembly forming an inner surface of the storage space, and including a fluid passage to guide cold air, which is generated from the evaporator, to the storage space, and a discharge port to discharge the cold air to the storage space. The fluid passage may include a first fluid passage to receive the cold air supplied from the evaporator, and a second fluid passage communicating with an outlet of the first fluid passage and split into a plurality of fluid passages. A deodorizing device may be provided in the first fluid passage to remove odor, and the cold air passing through the first fluid passage is split at the second fluid passage after passing through the deodorizing device.

The refrigerator according to the first aspect or to the second aspect may include one or more of the following features.

The plurality of fluid passages of the second fluid passage may include at least two fluid passages, say a first sub-passage (or first split-passage) and a second sub-passage (or second-split passage). The plurality of discharge ports includes at least a first discharge port and a second discharge port spaced apart from each other.

The first sub-passage guides air into the storage space from the first discharge port of the first and the second discharge ports, and the second sub-passage guides air into the storage space from the second discharge port of the first and the second discharge ports.

Optionally, the first sub-passage guides air into the storage space from only the first discharge port of the first and the second discharge ports, and the second sub-passage guides air into the storage space from only the second discharge port of the first and the second discharge ports.

Simply put, at least two of the plurality of fluid passages of the second fluid passage provide cold air into the storage space via different discharge holes.

The grill pan assembly may form at least a portion of a rear wall surface of the storage space.

The surface defining the storage space formed by the grill pan assembly may at least be a portion of a surface of a rear wall of the storage space.

In the present technique, the surface defining the storage space formed by the grill pan assembly has been depicted as the rear surface of the storage space. However, the storage space may have an open front surface which is opened and closed by the door, the rear surface facing the open front surface, two side surfaces extending from the open front surface to the rear surface, a top surface and a bottom surface. The surface defining the storage space formed by the grill pan assembly may also be one or more of - one or both of the side surfaces, the top surface, and the bottom surface.

The grill pan assembly may have a mounting port which is formed to be open in a front direction.

The deodorizing device may be inserted, e.g. reversibly or detachably insertable, into the fluid passage from an outside of the grill pan assembly through the mounting port.

The grill pan assembly may have a mounting port open into or to the storage space.

The deodorizing device may be reversibly or detachably insertable through the mounting port into the first fluid passage from an outside of the grill pan assembly, for example through the storage space into the mounting port.

The deodorizing device may include a front part exposed through a front surface of the grill pan assembly,

The deodorizing device may include a frame part protruding rearward from a rear surface of the front part and positioned inside the first fluid passage

The deodorizing device may include a deodorizing member mounted in the frame part. The cold air flowing in the first fluid passage may pass through the deodorizing member.

The deodorizing device may include a front part exposed through a front surface of the grill pan assembly facing the storage space, i.e. when inserted or suspended or provided in the first fluid passage.

The deodorizing device may include a frame part protruding rearward from a rear surface of the front part toward the first fluid passage (away from the storage space) and configured to be positioned or provided or suspended inside the first fluid passage.

The deodorizing device may include a deodorizing member configured to remove odor from the cold air and mounted in the frame part.

The deodorizing member may be a material which deodorized by adsorption and/or absorption and/or by chemically reacting and/or by physically binding or retaining from the cold air passing when passing through the deodorizing member odor particles/factors.

The deodorizing member may be provided in the first fluid passage in the flow path of the cold air flowing through the first fluid passage wherein the cold air flowing in the first fluid passage passes through the deodorizing member. In other words, the cold air is channeled to flow through the deodorizing member.

The deodorizing device and/or the deodorizing member may extend laterally i.e. side to side in the first fluid passage.

The deodorizing device and/or the deodorizing member may extend laterally i.e. side to side in the first fluid passage with respect to flow direction of the cold air.

The deodorizing device and/or the deodorizing member may extend across the first fluid passage such as to cover an entire cross-section (cross-section perpendicular to the flow direction of the cold air) or may cover only a part of the entire cross-section.

A mounting part may be recessed in the front surface of the grill pan assembly to receive the front part.

A mounting port may be formed inside the mounting part.

The frame part may pass through the mounting port.

The frame part may protrude a direction crossing a direction in which the first fluid passage extends.

A mounting part may be recessed in the surface of the grill pan assembly i.e. in the surface that is facing or defining the storage space. The mounting part may receive the front part, and may have a corresponding shape.

The mounting part may have a corresponding or complementary shape as that of the front part such that the front part may be received to fit (interference fitted) into the mounting part.

The mounting port may be formed inside the mounting part, and configured to receive into the frame part, i.e. when the front part is received into the mounting part, the frame part extends into or through the mounting port and is disposed or provided or suspended in the first fluid passage in the flow path of the cold air flowing therethrough.

The frame part may have a top surface and a bottom surface which are open and may be mounted such that a top surface and a bottom surface of the deodorizing member are exposed.

A plurality of holes may be formed, for example vertically extending, through the deodorizing member such that the cold air passes through the holes.

The frame part may have an open top surface and an open bottom surface, such that the deodorizing member mounted in the frame part is exposed to the first fluid passage toward downstream and upstream of the frame part, respectively.

Atop surface and a bottom surface of the deodorizing member may be exposed to an outside of the frame part through the top surface and the bottom surface of the frame part, respectively.

The deodorizing member may comprises a plurality of holes, i.e. through-holes, formed through the deodorizing member for allowing the cold air to pass through the deodorizing member.

The through holes may be formed vertically extending such that the flow direction of the cold air remains unchanged when flowing through an inlet to an outlet of the hole; or may be formed in a shape such that the flow direction of the cold air is unchanged (for example serpentine or zig-zag shaped or inclined holes) when flowing through an inlet to an outlet of the hole and thus causing greater contact with the deodorizing member.

The front part may be formed in a shape of a flat plate.

The frame part may protrude from the rear surface of the front part to pass through the front surface of the grill pan assembly.

The front part may be formed in a shape to be flush with the surface defining the storage space formed by the grill pan assembly, for example with the rear surface of the storage space.

Insulating materials or member(s) may be disposed at an upper portion and/or a lower portion of the frame part on the rear surface of the front part.

The grill pan assembly may include a grill plate forming a rear surface of the storage space, optionally having a shape of a flat plate, and having the discharge port.

The grill pan assembly may include a duct member provided on a rear surface of the grill plate and including the first fluid passage and the second fluid passage.

The grill pan assembly may include a grill plate forming the surface defining the storage space, and including the at least one or the plurality of discharge ports.

The duct member may be provided on a rear surface of the grill plate and include the first fluid passage and the second fluid passage.

The duct member may define an enclosed channel in which the first fluid passage and the second fluid passage are formed.

The duct member may be formed of an insulating material.

The duct member may include a deodorizing device receiving part which is recessed deeper than the first fluid passage and the second fluid passage to provide a space which is to receive the deodorizing device.

The duct member may includes a deodorizing device receiving part recessed deeper toward the storage space than the first fluid passage and/or the second fluid passage to provide a space for receiving the deodorizing device therein.

A cross-sectional area of the duct member at the deodorizing device receiving part may be greater than a cross-sectional area of the first fluid passage upstream, e.g. immediately upstream, of the deodorizing device receiving part and/or than a cross-sectional area of the first fluid passage downstream, e.g. immediately downstream, of the deodorizing device receiving part. As a result the flow of cold air through the deodorizing device receiving part, and thus through the deodorizing device is slowed down, providing greater time for interaction of the cold air and the deodorizing device or the deodorizing member of the deodorizing device.

A gradient surface, which has a height gradually reduced, as extending toward the deodorizing device, may be formed at a lower end of the deodorizing device receiving part.

The duct member may include a gradient surface formed at a lower end of the deodorizing device receiving part, wherein the gradient surface is inclined, preferably gradually inclined, from an immediately downstream part of the first fluid passage towards the deodorizing member receiving part such that cold air is directed by the gradient surface from the immediately downstream part of the first fluid passage toward the deodorizing device. Thus, ensuring a smooth flow of the cold air.

A sterilizing device configured to irradiate ultraviolet (UV) light toward the deodorizing device may be provided.

The sterilizing device may be provided inside the first fluid passage to irradiate ultraviolet (UV) light toward the deodorizing device.

The sterilizing device may include a substrate fixed to the duct member, and optionally disposed in parallel to the deodorizing member, and an UV light emitting diode (LED) disposed along the substrate to face the deodorizing member.

The sterilizing device may be disposed at an upper end of the deodorizing device receiving part and disposed to be spaced apart, e.g. vertically spaced apart, from the deodorizing device.

The storage space may be partitioned by a barrier and may include a freezing compartment, in which the evaporator is disposed, and a refrigerating compartment formed above the freezing compartment.

The grill pan assembly may be provided in the refrigerating compartment.

The duct member may further include a connection duct which protrudes downward to pass through the barrier and communicates with a space in which the evaporator is disposed, and the connection duct may communicate with an inlet of the first fluid passage.

A drawer may be disposed on a top surface of the barrier, and the connection duct may be formed to pass through a rear portion of the drawer.

A third fluid passage may be formed inside the connection duct to be connected with the inlet of the first fluid passage.

The connection duct may include a drawer discharge port which communicates with the third fluid passage, and supplies the cold air to the drawer.

The deodorizing device may be disposed above the drawer discharge port.

As described above, the refrigerator according to the present disclosure, has the following effects.

The refrigerator is capable of efficiently removing odor.

The refrigerator is capable of efficiently deodorizing cold air at one place and then splitting/distributing the deodorizing cold air and supplying the same to the storage space.

The deodorizing device may be mounted through the front surface of the grill pan assembly, thereby improving the assembly workability.

The deodorizing device has the structure directly exposed to the outside, when the door is open. Accordingly, the user may have the reliability of the operation for removing odor and destroying germs.

The deodorizing device may be inserted through the front surface of the grill pan assembly without the additional fluid passage and may be disposed on the fluid passage of the cold air, thereby improving productivity and reducing the manufacturing cost.

In particular, even in the model including the storage space having the narrower left-right widths the additional fluid passage for deodorizing is not required, thereby sufficiently ensuring the area of the fluid passage to prevent the shortage in the volume of the cold air or to prevent the cooling performance from being degraded.

The deodorizing device may be disposed on a single fluid passage to deodorize the entire portion of the cold air passing through the fluid passage. The fluid passage may be formed to split the cold air after the cold air passes through the deodorizing device, thereby maximizing the deodorizing performance and the sterilization performance. An additional deodorizing device is not required even in the split fluid passage structure, and the deodorizing performance may be sufficiently provided through one deodorizing device. Accordingly, the manufacturing cost may be reduced.

Further, the sterilizing device is provided above the deodorizing device to sterilize the deodorizing device and the air passing through the deodorizing device, thereby more improving the deodorizing performance while improving the purifying performance.

### BRIEF DESCRIPTION OF THE DRAWINGS

FIG. 1 is a front view illustrating a door, which is open, of a refrigerator according to an embodiment of the present disclosure;
FIG. 2 is a partially enlarged view illustrating an inner part of a refrigerating compartment;
FIG. 3 is a perspective view of a grill pan assembly according to an embodiment of the present disclosure;
FIG. 4 is a rear view of the grill pan assembly;
FIG. 5 is an exploded perspective view illustrating a coupling structure between a deodorizing device and a grill pan assembly according to an embodiment of the present disclosure;
FIG. 6 is a cross-sectional view taken along line VI-VI' of FIG. 3;
FIG. 7 is a perspective view of a deodorizing device, when viewed from a rear-upper portion of a refrigerator in which the deodorizing device is mounted;
FIG. 8 is a perspective view of a deodorizing device, when viewed from a rear-lower portion of a refrigerator in which the deodorizing device is mounted; and
FIG. 9 is a view illustrating cold air flowing in a grill pan assembly.

### DETAILED DESCRIPTION

Hereinafter, the detailed embodiment of the present disclosure will be described in detail with reference to accompanying drawings. The present disclosure may, however, be embodied in different forms and should not be construed as limited to embodiments set forth herein. Rather, these embodiments are provided so that the present disclosure will be thorough and complete, and will fully convey the scope of the present disclosure to those skilled in the art.

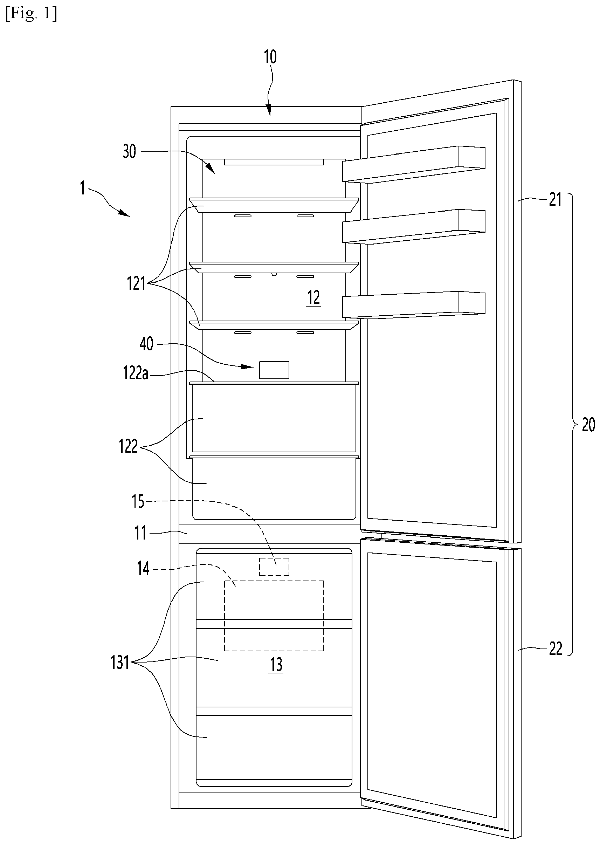

Before the description, directions will be defined below. According to an embodiment of the present disclosure, when viewed in FIG. 1, a direction of facing an entrance, which is open, of a storage space, a direction of facing an inner part of the storage space from a front surface, which is open, of an open door, a direction of facing a bottom surface on which a refrigerator is installed, and a direction of being away from the bottom surface may be defined as a front direction, a rear direction, a down direction, and an up direction, respectively.

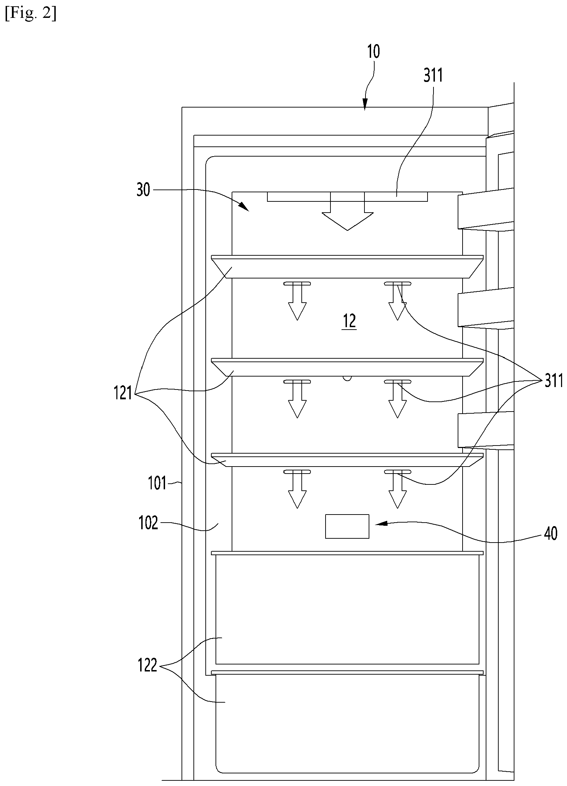

FIG. 1 is a front view illustrating a door, which is open, of a refrigerator according to an embodiment of the present disclosure. FIG. 2 is a partially enlarged view illustrating an inner part of a refrigerating compartment.

As illustrated in FIGS. 1 and 2, a refrigerator 1 according to an embodiment of the present disclosure may include a cabinet 10 having a storage space and a door 20 pivotably mounted at one side of the cabinet 10 to selectively open or close the storage space.

The storage space may be vertically partitioned by a barrier 11. An upper storage space may be formed above the barrier 11, and a lower storage space may be formed down the barrier 11. The upper storage space and the lower storage space may be controlled at mutually different temperatures to store foods at proper storing temperatures. For example, the upper storage space may be a refrigerating compartment 12, and the lower storage space may be a freezing compartment 13.

The refrigerating compartment 12 may include receiving members, such as a shelf 121 and a drawer 122, therein to receive foods. In addition and optionally, the drawer 122 may be provided at a lower portion of the refrigerating compartment 12, and may include a plurality of drawers 122 which are vertically stacked on each other. In addition and optionally, the drawer 122 may include a drawer cover 122a provided in the shape of a flat plate to shield a top surface, which is open, of the drawer 122.

The freezing compartment 13 may include a freezing compartment drawer 131 that is able to be introduced into or withdrawn from the inner part of the freezing compartment 13. A plurality of freezing compartments 13 may be consecutively arranged in a vertical direction.

The door 20 may include a refrigerating compartment door 21 pivotably mounted on the cabinet 10 to selectively open or close a storage space of the refrigerating compartment 12, and a freezing compartment door 22 pivotably disposed under the refrigerating compartment door 21 to selectively open or close a storage space of the freezing compartment 13.

Meanwhile, the cabinet 10 may include an outer case 101 constituting an outer portion thereof, and an inner case 102 provided inside the outer case 101 to form at least a portion of inner surfaces of the refrigerating compartment 12 and the freezing compartment 13. An insulating material (not illustrated) may be provided in a space between the outer case 101 and the inner case 102.

A rear wall surface of the refrigerating compartment 12 may be formed through a grill pan assembly 30. The grill pan assembly 30 may form the rear wall surface of the refrigerating compartment 12 and may include a fluid passage 330 to discharge cold air into the refrigerating compartment 12.

The grill pan assembly 30 may include a plurality of cold air discharge port 311. The cold air discharge port 311 may include an outlet communicating with the fluid passage 330 to discharge cold air, which is guided through the fluid passage 330, into the refrigerating compartment 12.

The cold air discharge ports 311 may be formed at positions corresponding to lower ends of a plurality of shelves 121 to uniformly cool spaces, which are partitioned by the shelves 121, by the cold air discharged from the cold air discharge ports 311. In addition and optionally, some of the cold air discharge ports 311 may be formed only at positions corresponding to some of the plurality of shelves 121.

A lower portion of the grill pan assembly 30 may have a structure communicating with a space, in which an evaporator 14 is disposed, to supply cold air, which is generated from the evaporator 14, into the refrigerating compartment 12. For example, the evaporator 14 may be provided at a rear portion of the freezing compartment 13 and may configured to supply the cold air to both the freezing compartment 13 and the refrigerating compartment 12 by one evaporator 14 and one blowing fan 15. In addition and optionally, although not illustrated, a suction fluid passage (not illustrated) may be further formed in a lower portion of the refrigerating compartment 12 and may communicate with the freezing compartment 13 to collect the air of the refrigerating compartment 12.

Hereinafter, the grill pan assembly 30 will be described in more detail with reference to accompanying drawings.

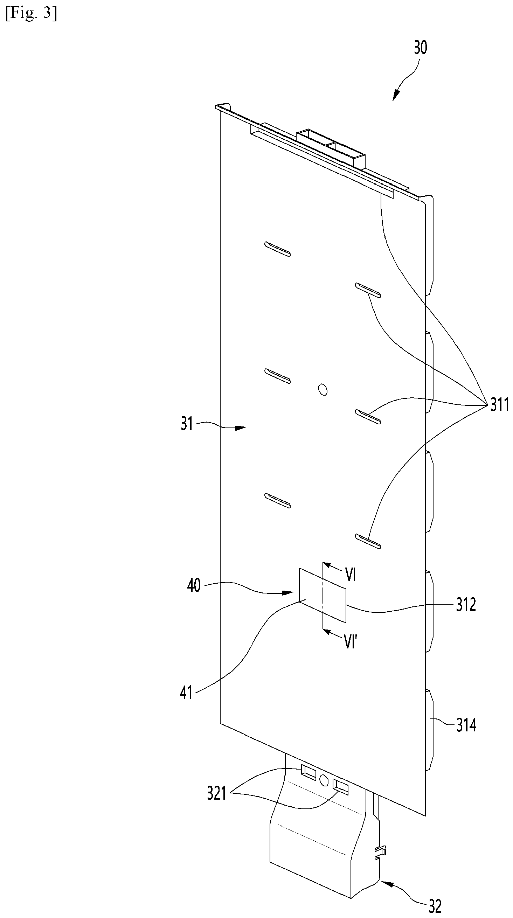

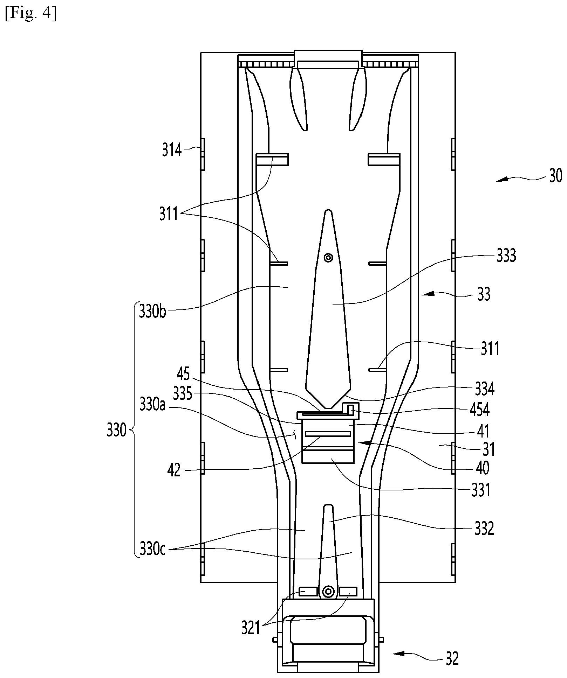

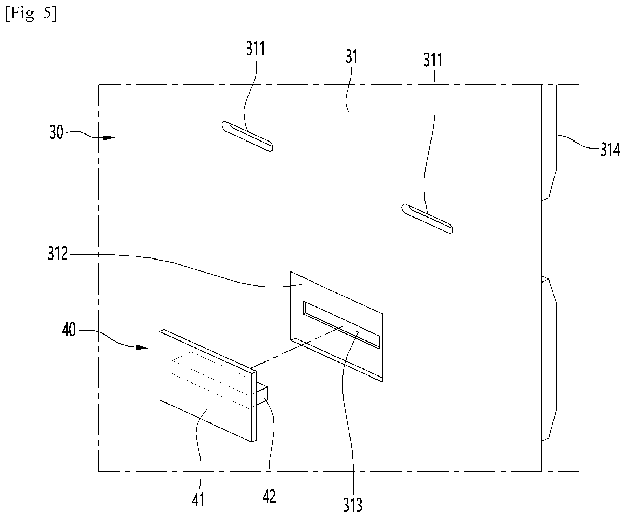

FIG. 3 is a perspective view of a grill pan assembly according to an embodiment of the present disclosure. FIG. 4 is a rear view of the grill pan assembly. FIG. 5 is an exploded perspective view illustrating a coupling structure between a deodorizing device and a grill pan assembly according to an embodiment of the present disclosure.

Although not illustrated, the grill pan assembly 30 may form at least a portion of the rear wall surface of the refrigerating compartment 12, and may be substantially provided in a rectangular shape. However, the present disclosure is not limited to the rectangular shape. For example, the grill pan assembly 30 may have various shapes, as long as the grill pan assembly 30 has the shape for constituting the rear wall surface of the refrigerating compartment 12.

The grill pan assembly 30 may include a grill plate 31 having the shape of a flat plate, and a duct member 33 provided on a rear surface of the grill plate 31 to form the fluid passage 330. In this case, the grill pan assembly 30 may include a single component formed by integrating the grill plate 31 with the duct member 33.

In detail, the grill plate 31 may be formed of a plastic material, and may form the rear surface of the refrigerating compartment 12. The grill plate 31 may be formed in the rectangular shape, and may have a size corresponding to the size of the rear wall surface of the refrigerating compartment 12.

The grill plate 31 may include a plurality of plate coupling parts 314 bent rearward and formed along the peripheral portion of the grill plate 31, and may be fixedly mounted on the inner case 102 by the plate coupling parts 314.

The plurality of cold air discharge port 311 may be formed in the grill plate 31. The cold air discharge ports 311 may be formed at positions corresponding to positions of the shelf 121, and may be formed in pair at left and right sides having equal heights.

In this case, the cold air discharge port 311 may be positioned in an inner area of the fluid passage 330, and may be positioned above the deodorizing device 40 to be described below. Accordingly, air deodorized and sterilized through the deodorizing device 40 may be discharged in the front direction through the cold air discharge port 311.

The upper most cold air discharge port 311 of the cold air discharge ports 311 may be positioned in an upper end of the grill plate 31, and may be provided in the form of one hole extending widthwise.

A mounting part 312 may be formed at the center of a lower portion of the grill plate 31 such that the deodorizing device 40 is mounted in the mounting part 312. The mounting part 312 may be recessed in a shape corresponding to the shape of a front surface of the deodorizing device 40, and a mounting port 313 may be formed through the center of the mounting part 312. The mounting port 313 may be open such that a portion of the deodorizing device 40 passes through the mounting port 313.

In detail, the deodorizing device 40 may include a front part 41 constituting a front surface of the deodorizing device 40 and a frame part 42 extending rearward from the front part 41. A deodorizing member 43 is received in the frame part 42. The frame part 42 may protrude rearward from the substantially central portion of the vertical height of the front part 41.

In this case, the size of the front part 41 may correspond to the size of mounting part 312. The front part 41 may be positioned inside the mounting part 312, in the state that the deodorizing device 40 is mounted on the grill plate 31. In addition and optionally, the frame part 42 may have the size corresponding to the size of the mounting port 313, when viewed in a cross-sectional view, and may be press-fitted into the mounting port 313, in the form of being locked to the circumference of the mounting port 313 and restricted to the mounting port 313.

Meanwhile, the mounting part 312 may be positioned under the lower most cold air discharge port 311 of the plurality of cold air discharge ports 311, and may be positioned in the lengthwise-center of the grill plate 31. In addition and optionally, the mounting part 312 may be positioned at a position at which the fluid passage 330 is split. In other words, the mounting part 312 may be positioned at a position of a single passage before the fluid passage 330 is split, and may be formed such that the fluid passage 330 is split above the mounting part 312.

A connection duct 32 may be provided at a lower end of the central portion of the grill plate 31 to protrude downward. The connection duct 32 may protrude downward to communicate with a space for the evaporator 14 disposed under the connection duct 32. Accordingly, when the blowing fan 15 operates, the cold air of the evaporator 14 may flow toward an upper portion of the grill pan assembly 30 through the connection duct 32.

Drawer discharge ports 321 may be additionally and optionally formed in an upper end of the connection duct 32. The drawer discharge port 321 may be positioned at a position corresponding to the drawer 122, and may be formed under the deodorizing device 40.

The drawer discharge port 321 allows some of the cold air, which is discharged from the evaporator 14, to be introduced into the drawer 122 disposed at the lowest portion of the refrigerating compartment 12 to cool the drawer 122. The drawer discharge port 321 may be positioned at a position close to the evaporator 14 such that the inner part of the drawer 122 has a sub-zero temperature or a temperature close to the sub-zero temperature. For example, the internal temperature of the drawer 12 may be maintained to be in the range of -1°C to 2°C. Germs may not be multiplied at such a low temperature, and odor is not made relatively. Accordingly, even if cold air is supplied without passing through the deodorizing device 40, the odor is not made inside the drawer 122. In other words, the drawer 122 may have the structure for directly introducing the cold air output through the evaporator 14, and may be maintained at a lower temperature as compared to the temperature of another area of an inner part of the refrigerating compartment 12.

A duct member 33 may be provided on a rear surface of the grill plate 31. The duct member 33 may include the fluid passage 330 formed in a rear surface of the grill plate 31, and may be formed of a material which is easily molded and has an excellent insulating property. For example, the duct member 33 may be formed of a styrofoam material.

The duct member 33 may be disposed the length-wise center of the grill plate 31, and may extend from the lower end of the grill plate 31 to the upper end of the grill plate 31. Accordingly, the cold air moving upward along the fluid passage 330 may pass through the deodorizing device 40, may be split, and may be supplied to the refrigerating compartment 12.

A lower end of the duct member 33 may be connected to the connection duct 32, and may extend to the upper end of the grill plate 31. In addition and optionally, the duct member 33 may be recessed in the front direction to form the fluid passage 330 which is a space for allowing the cold air to flow.

The fluid passage 330 may include a lower fluid passage 330a provided in the form of a single passage, and upper fluid passages 330b formed, as an upper end of the lower fluid passage 330a is split into a pair of left and right passages. The lower fluid passage 330a may be referred to as a first flow passage, and the upper fluid passage 330b may be referred to as a second flow passage.

A split guide 333 may be formed between the upper fluid passages 330b. In other words, the upper fluid passages 330b may be split into left and right fluid passages by the split guide 333. In addition and optionally, cold air discharge ports 311 may be formed along the split upper fluid passages 330b. In other words, the cold air flowing along the upper fluid passage 330b may be provided in the front direction through the cold air discharge port 311.

The center of a lower end of the split guide 333 may protrude downward, and gradient parts 334 may be formed to be more inclined upward in bi-lateral directions from the protruding center. Accordingly, the air output through the deodorizing device 40 may be guided to the inside of the upper fluid passages 330b, which are split into left and right fluid passages, through the gradient part 334.

A deodorizing device receiving part 335 to mount the deodorizing device 40 therein may be formed at the upper end of the lower fluid passage 330a. The deodorizing device receiving part 335 may be recessed in the front direction, and may be open to expose the grill plate 31. The deodorizing device receiving part 335 may be formed in size corresponding to the size of the deodorizing device 40. When the deodorizing device 40 is mounted in the grill pan assembly 30, the deodorizing device 40 may be exposed in the rear direction through the deodorizing device receiving part 335.

The widthwise length of the deodorizing device 40 and the widthwise length of the deodorizing device receiving part 335 may be formed in size corresponding to the widthwise length of the upper end of the lower flow passage 330a. Accordingly, the odor of the most part of cold air flowing upward through the lower fluid passage 330a may be removed while passing through the deodorizing device 40.

A light emitting diode (LED) module 45 may be mounted on the upper end of the deodorizing device receiving part 335. A LED module 45 may be configured to irradiate an ultraviolet ray toward the deodorizing device 40. Accordingly, the germs may be prevented from being multiplied in the deodorizing device 40, and germs in the air passing through the deodorizing device 40 may be sterilized. The LED module 45 may be referred to as a sterilizing device.

A gradient surface 331 may be formed at a lower end of the deodorizing device receiving part 335. The gradient surface 331 may guide cold air moving upward such that the cold air passes through the deodorizing device 40. The gradient surface 331 may have the structure of more protruding in the rear direction, as the gradient surface 331 extends downward from the lower end of the deodorizing device receiving part 335.

Accordingly, the cold air flowing upward along the lower fluid passage 330a moves toward the deodorizing device 40 after passing through the gradient surface 331, and is guided to pass through the deodorizing member 43 of the deodorizing device 40, such that germs and odor factors may be removed from the discharged cold air.

Meanwhile, a drawer fluid passage 330c may be formed in a lower portion of the duct member 33, and the fluid passage 330 may further include the drawer fluid passage 330c. In detail, a lower guide 332 may be further formed under a lower portion of the lower fluid passage 330a. The lower guide 332 may extend from the upper end of the connection duct 32 and may form a pair of drawer fluid passages 330c divided into left and right parts. In addition and optionally, each of the both divided drawer fluid passages 330c has a drawer discharge port 321 formed therein to supply cold air to the drawer 122. The drawer fluid passages 330c may be referred to as a third fluid passage.

Hereinafter, the structure of the deodorizing device 40 and the coupling structure of the deodorizing device 40 will be described in more detail with reference to accompanying drawings.

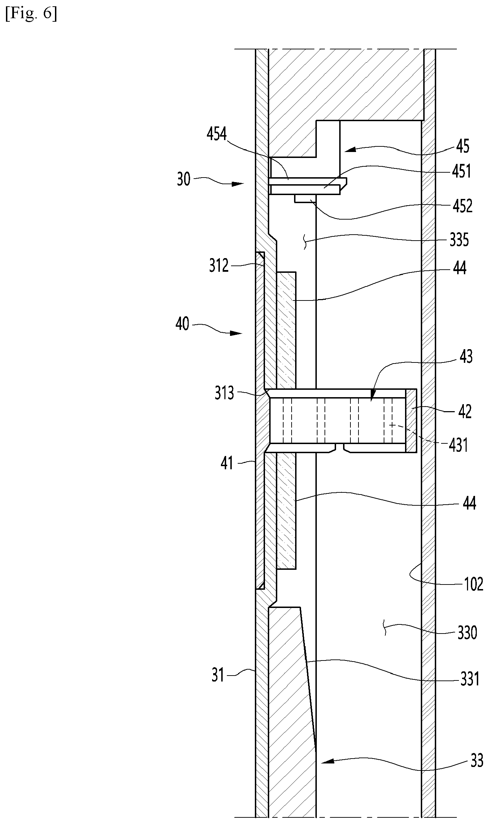

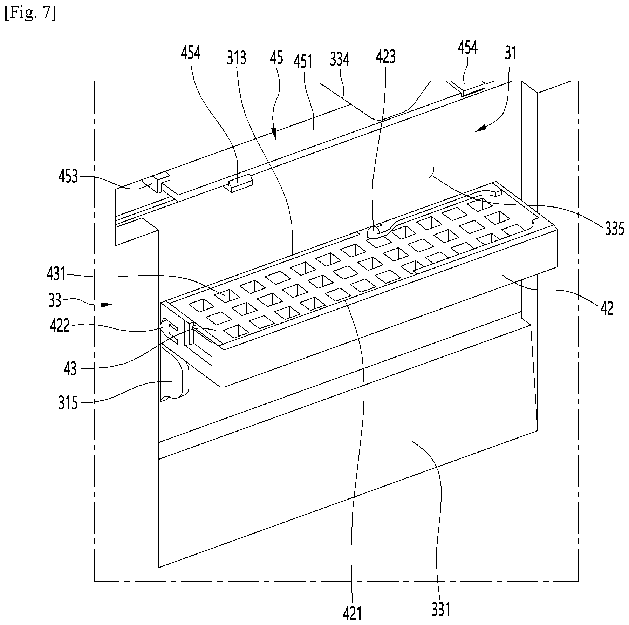

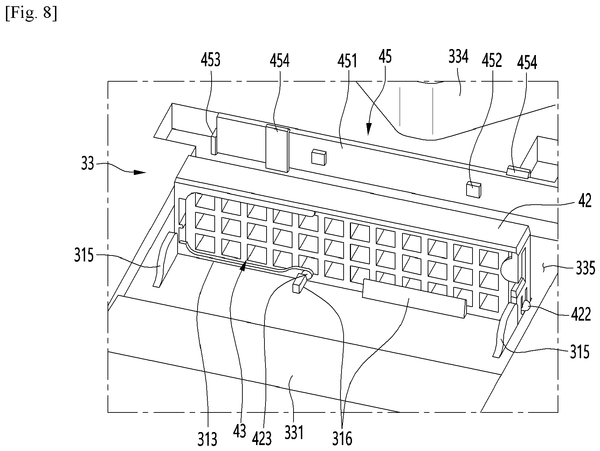

FIG. 6 is a cross-sectional view taken along line VI-VI' of FIG. 3. In addition, FIG. 7 is a perspective view of a deodorizing device, when viewed from a rear-upper portion of a refrigerator in which the deodorizing device is mounted. In addition, FIG. 8 is a perspective view of a deodorizing device, when viewed from a rear-lower portion of a refrigerator in which the deodorizing device is mounted.

Although illustrated in the drawings, the deodorizing device 40 may include the frame part 42 and the deodorizing member 43 inserted into and mounted in the frame part 42. The deodorizing device 40 may further include the front part 41 formed on the front end of the frame part 42. The front part 41 may be formed integrally with the frame part 42, and may be injection-molded with a plastic material. Accordingly, the structure in which the front part 41 and the frame part 42 are formed may be referred to a deodorizing case.

The front part 41 may be received into the mounting part 312 of the deodorizing device 40, and the frame part 42 may extend rearward through the mounting part 313. In addition, as a rear end of the frame part 42 extends to the inner case 102, when the grill pan assembly 30 is mounted, the space of the fluid passage 330 formed by the duct member 33 may be ensured.

The frame part 42 may protrude rearward from the front part 41 perpendicularly to the front part 41. In addition and optionally, the frame part 42 may be formed in a cuboid shape, in which a top surface and a bottom surface are open, and may be formed such that the deodorizing member 43 is received in the frame part 42.

The deodorizing member 43 may be formed of a material having excellent deodorization performance, and for example, may be formed of a material having an excellent adsorption property, such as charcoal, zeolite, or vermiculite, or a composite material. The deodorizing member 43 may be formed in the shape corresponding to the shape of the frame part 42, such that the deodorizing member 43 is received in the frame part 42. For example, the deodorizing member 43 may be formed in a hexahedron shape, and may be formed to have a top surface and a bottom surface wider than a circumferential surface. In addition and optionally, a plurality of holes 431 may be formed in the top surface and the bottom surface of the deodorizing member 43 to be open in the vertical direction through the deodorizing member 43.

The hole 431 may allow the air passing through the fluid passage 330 to pass in the vertical direction, such that an odor factor contained in the air is absorbed to the deodorizing member 43 while passing through the hole 431 in the vertical direction. In particular, the contact area between the deodorizing member 43 and the air may be increased by the hole 431, thereby improving the deodorizing performance. The air flowing through the fluid passage 330 passes through the deodorizing member 43 through the plurality of holes 431, thereby preventing the air from being lost while flowing.

Meanwhile, the frame part 42 may include first restricting protrusions 422 formed to protrude from left and right opposite surfaces of the frame part 42. The first restricting protrusions 422 may protrude outward to be locked to and restricted to left and right ends of the mounting port 313, when the deodorizing device 40 is mounted. The first restricting protrusions 422 may be formed to be inclined or rounded, and may be press-fitted into, locked to, and restricted to the mounting port 313, as the frame part 42 passes through the mounting port 313.

The frame part 42 may include second restricting protrusions 423 formed to protrude from upper and lower ends of the frame part 42. The second restricting protrusions 423 may protrude outward to be locked to and restricted to upper and lower ends of the mounting port 313, when the deodorizing device 40 is mounted. The second restricting protrusions 423 may be formed to be inclined or rounded, and may be press-fitted into, locked to, and restricted to the mounting port 313, as the frame part 42 passes through the mounting port 313.

The deodorizing device 40 may be securely mounted in the grill pan assembly 30 through the first restriction protrusion 422 and the second restriction protrusion 423. As the wider surface of the deodorizing member 43 crosses the airflow direction inside the fluid passage 330, the deodorizing performance may be ensured and the fluidity of the air may be prevented from being degraded.

Meanwhile, side support ribs 315 may protrude from left and right lower ends of the mounting port 313 to support opposite ends of the bottom surface of the frame part 42. The frame part 42 may be maintained to be in a mounting state of protruding perpendicularly to the rear surface of the grill plate 31 through the side support ribs 315.

A support part 316 may be further formed between the side support ribs 315. The support part 316 may protrude from a bottom end of the mounting port 313 to support the frame part 42 or the deodorizing member 43 under the frame part 42 or the deodorizing member 43 and may maintain the deodorizing device 40 to be in the more secure mounting state. The support part 316 is provided in the form of a protrusion or the form of a plate to stably support the deodorizing member 43.

Meanwhile, an insulating member 44 may be further provided on a rear surface of the mounting part 312 of the deodorizing device 40. Insulating members 44 are positioned above and under the frame part 42, and may be provided at a position corresponding to the position of the front part 41. The insulating member 44, which is to enhance insulating performance at an area of the mounting part 312 of the deodorizing device 40, may be attached to the rear surface of the grill plate 31 at an area in which the front part 41 is mounted. In other words, the insulating member 44 prevents dew condensation from being formed on the front part 41 or the front surface of the grill pan assembly 30 by cold air flowing along the rear surface of the mounting part 312 of the deodorizing device 40.

The LED module 45 may be mounted on the upper end of the deodorizing device receiving part 335. The LED module 45 may include a substrate 451, and a plurality of ultraviolet (UV) LEDs 452 mounted on the substrate 451. The plurality of UV LEDs 452 may be mounted along the bottom surface of the substrate 451, and may be configured to irradiate UV light toward the deodorizing member 43.

The substrate 451 may be formed to have a length correspond to the widthwise length of the deodorizing member 43 or to have a length more or less longer than the widthwise length of the deodorizing member 43, such that the UV light is uniformly irradiated throughout the entire portion of the deodorizing member 43. A first restriction part 453 and a second restriction part 454 may be formed on the rear surface of the grill plate 31, such that the substrate 451 may be mounted perpendicularly to the rear surface of the grill plate 31.

First restriction parts 453 may extend rearward from the rear surface of the grill plate 31 to correspond to left and right side portion of the substrate 451. The first restriction parts 453 may restrict the left and right side portions of the substrate 451 to prevent the substrate 451 from being shaken left or right, when the substrate 451 is mounted.

Second restriction parts 454 may extend rearward from the rear surface of the grill plate 31 to correspond to top and bottom surfaces of the substrate 451. In addition and optionally, the second restriction part 454 may make contact with the top surface and the bottom surface of the substrate 451. In addition and optionally, an end portion of the second restriction part 454 may be formed in a hook shape, such that the substrate 451 exactly maintains the mounting position thereof without being shaken in a front-rear direction.

A plurality of LEDs 452 may be arranged along the substrate 451, and may be arranged to face the top surface of the frame part 42, that is, the top surface of the deodorizing member 43. Accordingly, the UV light irradiated from the LED 452 may sterilize the air passing through the deodorizing member 43 and the hole 431 formed in the deodorizing member 43. Meanwhile, the LED 452 may have various structures to irradiate sterilizing light, and thus may be referred to as a light source or a light emitting member.

Meanwhile, the LED module 45 may be positioned inward of the deodorizing device receiving part 335, and may be formed to be prevented from more protruding rearward from the deodorizing device receiving part 335. Accordingly, the LED module 45 may be disposed not to interrupt air flowing along the fluid passage 330, and not to prevent cold air from being split by the split guide 333.

Hereinafter, the operation of the refrigerator 1 having the above structure will be described with reference to accompanying drawings.

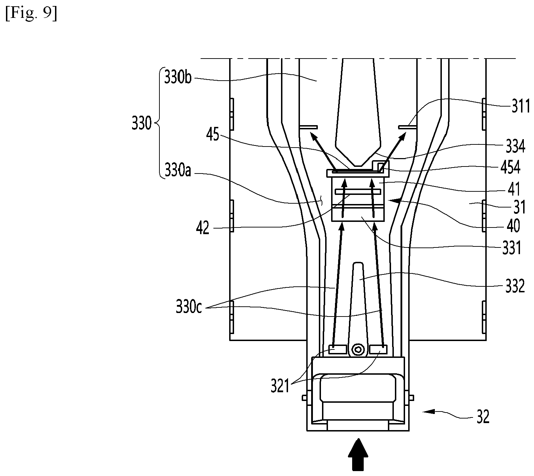

FIG. 9 is a view illustrating cold air flowing in a grill pan assembly.

Although illustrated in drawings, the cold air generated from the evaporator 14 may be supplied into the grill pan assembly 30 through the connection duct 32. The cold air introduced through the lower end of the grill pan assembly 30 may flow upward along the fluid passage 330 formed in the duct member 33.

In this case, the cold air, which flows upward along the lower fluid passage 330a, passes through the deodorizing device 40. In detail, the cold air flowing along the lower fluid passage 330a may be introduced into the deodorizing device receiving part 335 along the gradient surface 331. The most part of the air flowing upward along the lower fluid passage 330a by the frame part 42 and the deodorizing member 43, which are disposed inside the fluid passage 330 to cross the flowing direction of the air, flows upward through the hole 431 of the deodorizing member 43.

In this case, odor factors included in the air making contact with the deodorizing member 43 may stick to the deodorizing member 43. Accordingly, the odor may be removed from the air while the air is passing through the deodorizing member 43.

The LED module 45 may operate while the cold air is flowing along the fluid passage 330. The UV light irradiated from the LED 452 may destroy germs from the deodorizing member 43 and the air passing through the deodorizing member 43. The LED module 45 may be turned on at least while the blowing fan 15 is operated, and may operate while the cold air is flowing along the fluid passage 330, to sterilize the cold air through the LED 452.

The cold air deodorized and sterilized while passing through the deodorizing device 40 may be split into left and right parts through the gradient parts 334 of the split guide 333. In addition, the cold air may flow upward along the upper fluid passage 330b, and may be supplied to the refrigerating compartment 12 through the cold air discharge port 311 formed in the upper fluid passage 330b.

In other words, the entire portion of the cold air supplied to the refrigerating compartment 12 is deodorized and sterilized by the deodorizing device 40 and the LED module 45 while passing through the lower fluid passage 330a. In this state, the sterilized and deodorized air may be split and supplied to the upper fluid passage 330b, and supplied to the refrigerating compartment 12 through the cold air discharge port 311, thereby removing odor from the inner space of the refrigerating compartment 12 and preventing germs from being multiplied in the inner space of the refrigerating compartment 12.

## Claims

1. A refrigerator comprising:
a cabinet (10) having a storage space;
an evaporator (14) configured to generate cold air;
a grill pan assembly (30) forming a surface defining the storage space; and
a deodorizing device (40) to remove odor from the cold air;
wherein the grill pan assembly (30) includes:
a fluid passage (330) configured to guide cold air generated from the evaporator (14) to the storage space, and
at least one discharge port (311) to discharge the cold air from the fluid passage (330) into the storage space;
wherein the fluid passage (330) includes:
a first fluid passage (330a) for receiving the cold air from the evaporator (14); and
a second fluid passage (330b) communicating with an outlet of the first fluid passage (330a) for receiving the cold air from the first fluid passage (330a), wherein the second fluid passage (330b) comprises a plurality of fluid passages configured to split the cold air receiving from the first fluid passage (330a) and to guide the cold air so split to the storage space via the at least one discharge port (311);
wherein the deodorizing device (40) is provided in the first fluid passage (330a) in a flow path of the cold air flowing therethrough and upstream of plurality of fluid passages of the second fluid passage (330b).

2. The refrigerator of claim 1, wherein the surface defining the storage space formed by the grill pan assembly (30) is at least a portion of a surface of a rear wall of the storage space.

3. The refrigerator of claim 1 or 2, wherein the grill pan assembly (30) has a mounting port (313) open into the storage space, and
wherein the deodorizing device (40) is detachably insertable through the mounting port (313) into the first fluid passage (330a) from an outside of the grill pan assembly (30).

4. The refrigerator of any one of the preceding claims, wherein the deodorizing device (40) includes:
a front part (41) exposed through a surface of the grill pan assembly (30) facing the storage space;
a frame part (42) protruding from a rear surface of the front part (41) away from the storage space and configured to be positioned inside the first fluid passage (330a); and
a deodorizing member (43) configured to remove odor from the cold air and mounted in the frame part (42), and provided in the first fluid passage (330a) in the flow path of the cold air flowing through the first fluid passage (330a).

5. The refrigerator of claim 3 and 4, wherein a mounting part (312) is recessed in the surface of the grill pan assembly (30) facing the storage space, and configured to receive the front part (41), and
wherein the mounting port (313) is formed inside the mounting part (312) and configured to receive the frame part (42) to pass therethrough.

6. The refrigerator of claim 4 or 5, wherein the frame part (42) protrudes from the front part (42) in a direction crossing a direction in which the first fluid passage (330a) extends from the evaporator (14) towards the second fluid passage (330b).

7. The refrigerator of any one of claims 4 to 6, wherein the frame part (42) has an open top surface and an open bottom surface and,
wherein the deodorizing member (43) is mounted in the frame part (42) such that a top surface and a bottom surface of the deodorizing member (43) are exposed to an outside of the frame part (42) through the top surface and the bottom surface of the frame part (42), respectively, and/or
wherein the deodorizing member (43) comprises a plurality of holes (431) formed through the deodorizing member (43) for allowing the cold air to pass through the deodorizing member (43).

8. The refrigerator of any one claims 4 to 7, wherein the front part (41) is formed in a shape of a flat plate and/or wherein the front part (41) is formed in a shape to be flush with the surface defining the storage space formed by the grill pan assembly (30).

9. The refrigerator of any one of claims 4 to 8, wherein at least one insulating member (44) is disposed on the rear surface of the front part (41).

10. The refrigerator of any one of the preceding claims, wherein the grill pan assembly (30) includes:
a grill plate (31) forming the surface defining the storage space, and including the at least one discharge port (311); and
a duct member (33) provided on a rear surface of the grill plate (31) and including the first fluid passage (330a) and the second fluid passage (330b).

11. The refrigerator of claim 10, wherein the duct member (33) includes a deodorizing device receiving part (335) recessed deeper toward the storage space than the first fluid passage (330a) and/or the second fluid passage (330b) to provide a space for receiving the deodorizing device (40) therein.

12. The refrigerator of claim 11, wherein the duct member (33) includes a gradient surface (331) formed at a lower end of the deodorizing device receiving part (335), wherein the gradient surface (331) is inclined such that cold air is directed by the gradient surface (331) toward the deodorizing device (40).

13. The refrigerator of any one of the preceding claims, comprising a sterilizing device (45) configured to irradiate ultraviolet (UV) light toward the deodorizing device (40).

14. The refrigerator of any one of the preceding claims, wherein the storage space is partitioned by a barrier (11) and includes a freezing compartment (13) and a refrigerating compartment (12)
wherein the evaporator (14) is disposed at the freezing compartment (13), and wherein the grill pan assembly (30) is provided at the refrigerating compartment (12).

15. The refrigerator of any one of the preceding claims, further including:
a connection duct (32) communicating with a space in which the evaporator (14) is disposed, and
wherein the connection duct (32) communicates with an inlet of the first fluid passage (330a) to guide cold air from the evaporator (14) into the first fluid passage (330a).
